# EUROPEAN PATENT SPECIFICATION

(11) **EP 3 644 968 B1**
(45) Date of publication and mention of the grant of the patent: **08.01.2025**
(21) Application number: 17780234.5
(22) Date of filing: 29.06.2017
(51) Int. Cl.: A61K 9/00, A61K 9/10, A61K 47/14, A61K 47/36

(54) **LEVOCLOPERASTINE FENDIZOATE SUSPENSION HAVING ENHANCED DISSOLUTION AND RESUSPENDABILITY**
LEVOCLOPERASTIN-FENDIZOAT-SUSPENSION MIT VERBESSERTER AUFLÖSUNG UND RESUSPENDIERBARKEIT
SUSPENSION DE FENDIZOATE DE LÉVOCLOPERASTINE PRÉSENTANT UNE DISSOLUTION ET UNE CAPACITÉ DE REMISE EN SUSPENSION AMÉLIORÉES

(43) Date of publication of application: 06.05.2020
(73) Proprietor: Ilko Ilaç Sanayi Ve Ticaret Anonim Sirketi, Sancaktepe/Istanbul (TR)
(72) Inventor: ÖNCEL, Hatice, Sancaktepe/Istanbul (TR); ÇAPAN, Yilmaz, Ankara (TR); PINARBASLI, Onur, Ankara (TR); AKANSEL, Sibel, Ankara (TR); SARRAÇOGLU, Nagehan, Ankara (TR)
(74) Representative: Bulut, Pinar
(86) International application number: PCT/TR2017/050290
(87) International publication number: WO 2019/004953

(56) References cited:
- EP-A1- 0 385 491
- EP-A1- 0 894 794
- EP-A1- 0 894 794
- US-A1- 2017 128 379
- P. ALIPRANDI ET AL: "Therapeutic Use of Levocloperastine as an Antitussive Agent : An Overview of Preclinical Data and Clinical Trials in Adults and Children", CLINICAL DRUG INVESTIGATION, vol. 22, no. 4, 1 April 2002 (2002-04-01), NZ, pages 209 - 220, XP055451980, ISSN: 1173-2563, DOI: 10.2165/00044011-200222040-00001
- B SHARMA ET AL: "Development and Validation of RP-HPLC Method for Simultaneous Estimation of Levocloperastine Fendizoate and Chlorpheniramine Maleate in their Combined Dosage Form", AUSTIN J ANAL PHARM CHEM, 19 April 2017 (2017-04-19), XP055451970, Retrieved from the Internet <URL:http://austinpublishinggroup.com/analytical-pharmaceutical-chemistry/download.php?file=fulltext/ajapc-v4-id1083.pdf> [retrieved on 20180216]

## Description

### Technical field:

The invention relates to formulation of a stable oral pharmaceutical suspension composition with improved dissolution and resuspendability properties comprising a therapeutically effective amount of water-insoluble drug substance levocloperastine or a pharmaceutically acceptable salt thereof, having volume particle size distribution D₉₀ less than 100 µm and 0.25% (w/v) to 0.36% (w/v) of xanthan gum as a suspending agent, 0.25% (w/v) of polyoxyethyl stearate as a surfactant and one or more pharmaceutically acceptable excipients.

### Prior Art:

Levocloperastine is the levorotatory isomer of DL-cloperastine with a molecular formula of C₂₀H₂₄ClNO, chemical name of which is 1-[2-[(4-Chlorophenyl) phenylmethoxy] ethyl] piperidine. It was first introduced at 1972 in Japan, and then in Italy at 1981. Pharmaceutical-grade levocloperastine exists as a salt, typically in the form of levocloperastine hydrochloride or levocloperastine fendizoate. In this present invention, a liquid oral dose form preferably suspension dose of levocloperastine fendizoate is studied. The structural formula of Levocloperastine fendizoate is represented below (Formula I);

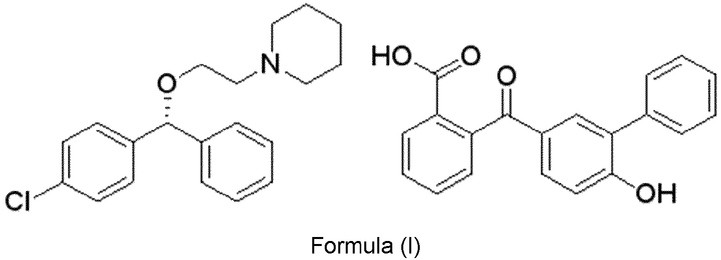

Cloperastine was first studied by Takagi et al at the University of Tokyo. This research group investigated the pharmacological activity of several derivatives of diphenhydramine, an antihistamine substance, and discovered that cloperastine had the greatest efficacy against cough caused by tracheal mechanical stimulus in the guinea pig. This effect was 1.9 times greater than codeine without any narcotic effects. Cloperastine, which also possesses broncho relaxant and antihistaminic activity, helped to relax the bronchial musculature which had become tight due to stimulation by acetylcholine and histamine.

EP 0894794 A1 discloses levorotatory enantiomer L(-) cloperastine and its salts (e.g., hydrochloride - fendizoate), which has made it possible to obtain a more active, less toxic product having fewer side effects compared to the D(+) isomer and the racemic mixture currently available on the market. In the clinical field, this means a better tolerability and safety of use of the drug.

Cough is a normal mechanism for the maintenance of a healthy respiratory system. It is characterized by an initial, short inspiratory phase, followed by closure of the glottis and rapid expiration with violent expulsion of air. In general, it is an important defense mechanism that expels infectious or excessive secretions from the tracheobronchial tree, and removes and dislocates exudates, cellular detritus, and foreign bodies.

A pharmacological and clinical overview of cloperastine in the treatment of cough indicated that this compound can be safely used in a wide section of population (children, adolescents and adults), according to the indications on the labelling and appropriate medical evaluation. The initial response at the therapeutic dose range (10-20 mg three times daily for adults) is 20-30 min after oral administration. Moreover, the duration of action of a single dose of cloperastine is 3-4 h.

Symptomatic treatment with antitussives is usually employed if the cough is nonproductive, barking, and significantly interferes with the well-being of the pediatric or adult patient (causing, eg, nausea, vomiting, insomnia, and/or headache). Antitussives can be divided into two main categories depending upon whether the mechanism of action is central or peripheral. Central antitussives include narcotics and non-narcotics, and act more or less selectively on the cough center located in the medulla oblongata. Non-narcotic therapy is preferable in children, because narcotic antitussives (eg, levorphanol, codeine) may cause respiratory center depression, nausea, vomiting, constipation, addiction, and physical dependency.

Levocloperastine is an antitussive agent that acts both centrally, on the bulbar cough center, and peripherally, on the cough receptors in the tracheobronchial tree. It is also endowed with an antihistaminic (sharing an ethylamine moiety with H₁ receptor antagonists) and papaverine-like activity similar to codeine but without its narcotic effects. This dual mechanism of action makes it very effective in the treatment of cough associated with many chronic and acute conditions in patients of all ages. Pharmacological studies have shown that the molecule acts on the cough center without depressing the respiratory center, and that it has no negative cardio circulatory effects.

The pharmaceutical preparations of levocloperastine may be in solid form (such as capsules, tablets, sugar-coated pills with immediate or delayed action, single-dose packets, etc.), in liquid form (solutions or suspensions ready for use or to be prepared as required); suppositories and injectable solutions. Pharmaceutical compositions administered in solid form are usually intended to be swallowed whole. Children, older persons and many other persons including disabled or incapacitated patients often have trouble swallowing tablets or capsules. For many such patients, including pediatric and geriatric patients, a liquid dose form is preferable because of the ease application. In this present invention, a liquid oral dose of levocloperastine fendizoate suspension form is preferred.

Suspensions are a two-phase system having solid substantially water insoluble active agent particles dispersed throughout liquid medium. A suspension does not encompass emulsions, which are meant to describe liquids suspended within liquid carriers or syrup formulations containing only substantially fully dissolved pharmaceutical active agents.

The suspension form must be physically stable (no appreciable settling) for a sufficient time, chemically stable over the required time (shelf-life), possess a viscosity that allows it to be used for its intended purpose, be easily reconstituted (redispersible) upon shaking, easy to manufacture and be acceptable in use to the patient, care-giver or other user.

Milani M. (2012) discloses clinical efficacy of Levocloperastine by giving several clinical trials conducted both in adult and in children. Clinical trials show that Levocloperastine can produce a greater reduction in the intensity and frequency of cough compared with other antitussive compounds. It is an effective antitussive agent for the treatment of cough in patients of all age groups. It has a more rapid onset of action than standard agents with an improved tolerability profile. Levocloperastine represents a valuable alternative to currently used antitussive agents with the added advantage of faster onset of action and improved tolerability.

P. Aliprandi et al (2002) discloses an oral suspension comprising levocloperastine fendizoate. It is silent about the formulation and particularly particle size distribution of the active substances. It is aimed to show that levocloprastin represents effective alternative to currently used antitussive agents with the advantage of faster onset of action and better tolerability in all patient groups.

Although pharmaceutical dosage form of suspension is known, there is not enough information about levocloperastine fendizoate suspension dosage form, especially undesirable properties of agglomeration/ phase separation and resuspendability, particularly with a pharmaceutical active substance practically insoluble in water. More importantly, the resuspendability properties of suspensions directly affect content uniformity and accurate dosing.

The insolubility property of levocloperastine fendizoate presents a formulary challenge during product development of an aqueous liquid oral preparation. Hence, in this invention it would be desirable to formulate physically stable levocloperastine fendizoate suspension dosage form with reduced propensity for occurrence of irreversible agglomeration and/or phase separation and improved resuspensibility property of drug product that is suitable for the suspension of pharmaceutical actives substantially insoluble in water.

### Description of the Invention:

An object of the present invention is to provide pharmaceutical suspension compositions as disclosed in the claims, comprising a therapeutically effective amount of water-insoluble drug substance, that is levocloperastine fendizoate, and at least one inactive ingredient, which demonstrate reduced propensity for occurrence of irreversible agglomeration and/or phase separation.

Another object of the present invention is to provide pharmaceutical suspension compositions as disclosed in the claims, comprising a therapeutically effective amount of water-insoluble drug substance, that is levocloperastine fendizoate, and at least one inactive ingredient, which demonstrate improved drug dissolution rates and resuspendability properties as compared to the commercially available reference product.

The invention is using levocloperastine fendizoate drug substance having volume particle size distribution D₉₀ of less than 100 µm as measured by laser diffraction.

The invention is directed to an oral pharmaceutical suspension composition comprising: (i) a therapeutic effective amount of levocloperastine fendizoate having a volume particle size distribution D₉₀ of less than 100 µm as measured by laser diffraction;(ii) 0.25% (w/v) to 0.36% (w/v) of xanthan gum as a suspending agent;(iii) 0.25% (w/v) of polyoxyethyl stearate as a surfactant; and(iv) one or more pharmaceutically acceptable excipients.

A suspension formulation according to the present invention is particularly suitable for ease of dosing in children, and in adults having problems with swallowing capsules and tablets.

Suspension dosage form is a preferred and widely accepted dosage forms for insoluble or poorly soluble drugs for various therapeutic applications. However, when a suspension of active substance is used there is the problem that the homogeneity of the suspension has to be ensured to ensure accurate dosing. A suspension requires adequate shaking of the container to resuspend the drug uniformly before dosing. Difficult redispersion of the drug from a sediment, or in the worst case, from caking, will result in under- and overdosing.

Therapeutically effective amount means providing sufficient amount of a substance that is non-toxic to a subject to achieve the desired therapeutic effect. The term therapeutically effective amount will be understood by the skilled adressee to include a range of amounts that will vary according to the condition to be treated, its severity and the health profile and status of the receipt subject. The exact amount would be able to determine by the skilled adressee.

According to the definitions of solubility as recited in the USP reproduced as Table 1 below, interpretation of the term `practically insoluble or insoluble' refers having a solubility of about more than 10.000 parts of solvent required to solubilize one part of drug. If water is the solvent, then the water solubility of an 'insoluble' drug substance can be less than 0.1 mg/mL. The water solubility of Levocloperastine fendizoate is lower than 0.1 mg/mL, therefore it is classified as water-insoluble. Because of this property, dissolution of the product & bioavailability is always a challenge to develop the Levocloperastine fendizoate product in formulation.

**Table 1 USP solubility definitions**

| Descriptive terms | Parts of solvent needed for 1 part solute |
|---|---|
| Very soluble | < 1 |
| Freely soluble | 1-10 |
| Soluble | 10-30 |
| Sparingly soluble | 30-100 |
| Slightly soluble | 100-1000 |
| Very slightly soluble | 1000-10,000 |
| Practically insoluble or insoluble | > 10,000 |

In suspensions, the physical characteristics of the fluid and the size of particles both have an effect on precipitation and aggregation. In practice, finer particles generally make for a more stable suspension. Particle size also affects the behavior of a formulation during processing and, ultimately, its content uniformity. Of course, the particle size distribution will also have a direct influence on the texture and feel of the finished product in terms of both efficacy and consumer perception. The particle size of levocloperastine fendizoate, in combination with other factors such as temperature, and composition viscosity, influence the tendency of the particles to aggregate, settle, and unevenly disperse throughout the pharmaceutical composition. Aggregation of insoluble levocloperastine fendizoate particles changes the release profile of the drug.

Reducing the particle size is a way of slowing sedimentation. However, small particles tend to cake more severely because of the increased surface energy from the larger surface area, making redispersion much more difficult and sometimes impossible.

Small particle size is desirable for reasons other than slowing the rate of sedimentation. For drugs that are not very soluble, smaller particles generally dissolve faster due to the increase in the total surface area, which in turn enhance bioavailability. Also, smaller drug particles are less likely to cause grittiness, which improves the palatability of the finished product.

The invention provides a pharmaceutical levocloperastione fendizoate suspension composition which has physical stability and good uniformity even with gentle shaking during and after production.

Particle size is determined by laser light scattering using a particle size analyzer, such as the proprietary Mastersizer^{™} apparatus in a dry dispersion system available from Malvern Instruments Ltd. (Malvern Mastersizer-2000, single narrow mode). Laser diffraction enables to measure particle size distributions by measuring the angular variation by scattering light intensity as a laser beam passes through a dispersed particulate sample. Large particles scatter light at small angles relative to the laser beam and small particles scatter light at large angles. The angular scattering intensity data is then analyzed to calculate the size of the particles responsible for creating the scattering pattern, using the Mie theory of light scattering. The particle size is reported as a volume equivalent sphere diameter.

A common approach to define the distribution width is to cite three values on the x-axis as D₁₀, D₅₀ and D₉₀. The D₅₀, named as median, has been defined as the diameter where 50 percent of the distribution lies below this value. Similarly, D₉₀ has been defined as 90 percent of the distribution lies below, and 10 percent of the population lies below the D₁₀.

According to the invention, there is a therapeutic effective amount of suspended levocloperastine or a pharmaceutically acceptable salt thereof wherein levocloperastine fendizoate drug substance has volume particle size distribution D₉₀ of less than 100 µm preferably less than 50 µm.

Particle size distribution results of levocloperastine fendizoate used in the invention is given below.

| | | |
|---|---|---|
| d(0.1): 3.031 µm | d(0.5): 13.126 µm | d(0.9): 39.322 µm |

The desired particle size range material is obtained directly from a synthesis process or alternatively any known particle size reduction processes can be used, such as but not limited to sifting, milling, micronization, fluid energy milling, ball milling and the like.

As embodied and fully described herein the present invention provides a pharmaceutical suspension composition comprising,
(a) a therapeutically effective amount of water-insoluble drug substance of levocloperastine fendizoate, having volume particle size distribution D₉₀ less than 100 µm, preferably D₉₀ less than 50 µm as measured by laser diffraction,
(b) a suspending system consisting essentially 0.25% (w/v) to 0.36% (w/v) of xanthan gum as a suspending agent,
(c) 0.25% (w/v) of polyoxyethyl stearate as a surfactant,
(d) an effective amount of water; and
(e) an effective amount of sweetener composition to provide a palatable taste.

According to FDA, an immediate release drug product is considered 'rapidly dissolving' when 85 % or more of the labeled amount of the drug substance dissolves within 30 minutes. Also, an immediate release product is considered 'very rapidly dissolving' when 85 % or more of the labeled amount of the drug substance dissolves within 15 minutes.

The pharmaceutical levocloperastine fendizoate suspension composition according to the present invention exhibits a dissolution profile such that about 85% of the levocloperastine fendizoate is released within 15 minutes; and wherein the release rate is measured in USP Apparatus 2 (paddle, 50 rpm) using 900 ml of 0.1 N HCl at 37°C ±0.5°C.

The inventive composition further comprises pharmaceutically acceptable excipients. Suitable excipients for the production of suspension include surfactant, suspending agent, preservatives, sweeteners, flavoring agents, antifoaming agent, taste masking agent, pH adjustment agent and solvent.

Suspending agents are used to prevent sedimentation by affecting the rheological behavior of a suspension. An ideal suspending agent should have certain attributes: (1) it should produce a structured vehicle, (2) it should be compatible with other formulation ingredients, and (3) it should be nontoxic. Generally used suspending agents in suspension include cellulosic derivatives (methylcellulose, carboxymethyl cellulose, hydroxyethyl cellulose, and hydroxypropyl methylcellulose), synthetic polymers (carbomers, polyvinylpyrrolidone poloxamers, and polyvinyl alcohol), and polysaccharides and gums (alginates, xanthan, guar gum, etc.). In the present invention, xanthan gum which is a high molecular weight polysaccharides gum is suitable for use. Xanthan gum was preferred as suspending agent and stabilizer in dispersing medium due to its acceptable toxicological and safety properties for food and pharmaceutical applications. It is soluble in water and imparts its high viscosity at low concentration with thixotropic flow characteristics.

Surfactant is a general name for materials that possess surface activity. There are several general classes of surfactants: anionic, cationic, amphoteric and non-ionic. The largest group of surfactants used in the formulation of pharmaceutical suspensions is the non-ionic surfactants. Types of non-ionic surfactants used in pharmaceutical suspensions are polyethylene glycol fatty acid esters, such as polyoxyethyl stearate, polyethylene glycol 400 stearate, polyethylene glycol 2000 stearate. In the present invention polyoxyethyl stearate is used. Inclusion of a surfactant increases the solubility and wettability of the drug particles. Advantageously, the use of high molecular weight polyoxyethylstearate increases solubility of relatively low solubility therapeutic agents.

Preservative refers to antimicrobial agents used to suppress the growth of microorganisms in the product of pharmaceutical formulations. Suitable preservatives include one or more of sodium benzoate, benzoic acid, ethylene diamine tetraacetic acid, sorbic acid, bronopol, butyl paraben, methyl paraben, ethyl paraben, propyl paraben, sodium propionate, chlorhexidine, potassium sorbate, propylene glycol, sodium bisulfite, sodium metabisulfite, sodium salts of hydroxybenzoate and the like. In the present invention combination of methyl paraben and propyl paraben is used.

Suitable sweeteners include one or more of sucrose, sorbitol, xylitol, dextrose, fructose, maltitol, acesulfame potassium, aspartame, saccharin, saccharin sodium, liquid maltitol, liquid glucose, cyclamate, sodium cyclamate and the like. In the present invention xylitol is used.

Suitable flavoring agents include one or more of artificial strawberry flavor, artificial cream flavor, vanilla, cherry, raspberry banana and the like. In the present invention banana flavor is selected.

Suitable solvents include one or more of water, glycerol, propylene glycol, polyethylene glycol, ethanol and the like.

The pH of the formulation is inherently provided by the excipients present in the formulation; alternatively, a pH adjustment agent is employed. In the present invention no pH adjustment agent is used, the final pH is provided by the excipients present in the formulation.

Unless otherwise indicated, percentages provided for the ingredients of the suspension of the present invention are weight/volume (w/v) percentages. Those weight/volume percentages are in units of grams per milliliter.

The following examples are provided to illustrate the invention and are not intended to be limiting thereof:

### Example 1.

In this example levocloperastine fendizoate drug substance having particle size distribution (D₉₀) less than 100µm is used.

Particle size distribution results of levocloperastine fendizoate used in this example is given below.

| | | |
|---|---|---|
| d(0.1): 3.031 µm | d(0.5): 13.126 µm | d(0.9): 39.322 µm |

| **Levocloperastine Fendizoate 708 mg/100 mL Suspension** | **% amount in unit dose (%w/v)** |
|---|---|
| Levocloperastine Fendizoate | 0.71 |
| Methyl paraben | 0.1 |
| Propyl paraben | 0.05 |
| Xanthan gum | 0.36 |
| Polyoxyethyl stearate | 0.25 |
| Xylitol | 32.0 |
| Banana flavor | q.s. |
| Deionized Water | q.s. |

Propyl paraben and methyl paraben are dissolved in water. While heating the solution, xylitol and polyoxyethyl stearate are added until a clear solution is obtained. Levocloperastine fendizoate drug substance, xanthan gum and banana flavor are added and suspended with stirring. The suspension is made up to volume with water and it is passed through the homogenizer. Finally finished product filled into glass bottles.

### Example 2.

In order to examine the effect of particle size distribution (D₉₀) of levocloperastine fendizoate drug substance on drug release, same production method of Example 1 is performed with different particle size distribution (D₉₀) of levocloperastione fendizoate drug substance.

In this example levocloperastine fendizoate drug substance having particle size distribution (D₉₀) higher than 150µm is used.

Particle size distribution results of levocloperastine fendizoate used in this example is given below.

| | | |
|---|---|---|
| d(0.1): 8.148 µm | d(0.5): 51.879 µm | d(0.9): 156.215 µm |

### Example 3.

In order to examine the effect of xanthan gum amount in unit dose on drug release, same production method of Example 1 (levocloperastine fendizoate drug substance having particle size distribution (D₉₀) of less than 100 µm) is performed with low xanthan gum amount ratio.

| **Levocloperastine Fendizoate 708 mg/100 mL Suspension** | **% amount in unit dose (%w/v)** |
|---|---|
| Levocloperastine Fendizoate | 0.71 |
| Methyl paraben | 0.1 |
| Propyl paraben | 0.05 |
| Xanthan gum | 0.25 |
| Polyoxyethyl stearate | 0.25 |
| Xylitol | 32.0 |
| Banana flavor | q.s. |
| Deionized Water | q.s. |

Propyl paraben and methyl paraben are dissolved in water. While heating the solution, xylitol and polyoxyethyl stearate are added until a clear solution is obtained. Levocloperastine fendizoate active substance, xanthan gum and banana flavor are added and suspended with stirring. The suspension is made up to volume with water and it is passed through the homogenizer. Finally finished product filled into glass bottles.

### Example 4.

In order to examine the effect of Polyoxyethyl stearate amount in unit dose on drug release, same production method of Example 1 (levocloperastine fendizoate drug substance having particle size distribution (D₉₀) of less than 100 µm) is performed with low Polyoxyethyl stearate amount ratio.

| **Levocloperastine Fendizoate 708 mg/100 mL Suspension** | **% amount in unit dose (%w/v)** |
|---|---|
| Levocloperastine Fendizoate | 0.71 |
| Methyl paraben | 0.1 |
| Propyl paraben | 0.05 |
| Xanthan gum | 0.36 |
| Polyoxyethyl stearate | 0.06 |
| Xylitol | 32.0 |
| Banana flavor | q.s. |
| Deionized Water | q.s. |

Propyl paraben and methyl paraben are dissolved in water. While heating the solution, xylitol and polyoxyethyl stearate are added until a clear solution is obtained. Levocloperastine fendizoate active substance, xanthan gum and banana flavor are added and suspended with stirring. The suspension is made up to volume with water and it is passed through the homogenizer. Finally finished product filled into glass bottles.

### Determination of precipitation in suspension samples

The prepared suspensions (Example1-4) were packaged in a glass bottle and left as such for 6 hours. Then, the presence of a precipitate in the suspension at the bottom of the bottles was visually observed. Any precipitate was not observed after 6 hours duration for the prepared suspensions (Example 1-4).

### Determination of resuspendability of suspension samples

Resuspendability is the ability to resuspend the settled particles with a minimum amount of shaking after a suspension has sedimented on standing for some time. The resuspendability of the suspensions was evaluated quantitatively by using content uniformity evaluation. In order to verify the uniformity of dose of the obtained formulations, uniformity of content test was performed according to pharmacopoeias guidance. Suspension content uniformity must be controlled in order to administer an accurate and uniform dose.

According to USP-pharmacopoeia, the general criteria for acceptable levels of drug content uniformity in suspensions are not less than 90.0% and not more than 110.0% of the labelled amount.

**Procedure:** The prepared suspensions (Example 1-4) and commercial reference product were allowed to sediment in stoppered glass bottles for 1 month. All samples were redispersed by shaking equal time (15 seconds) with the same manner. A certain amount of sample is withdrawn from different heights (10 times for each bottle) and content uniformity (assay) determination of levocloperastine fendizoate analysis is performed. The content uniformity evaluation results are given below in Table 2. Relative standard deviation (RSD%) was calculated according to pharmacopoeias guidance.

**Table 2. Content uniformity evaluation assay results of Levocloperastine fendizoate**

| **Sample No** | **Levocloperastine fendizoate %** | | | | |
|---|---|---|---|---|---|
| | **Reference Product** | **Example 1** | **Example 2** | **Example 3** | **Example 4** |
| **1** | 92.1 | 99,8 | 92,5 | 99,8 | 91,5 |
| **2** | 88.6 | 98,2 | 96,5 | 98,9 | 105,9 |
| **3** | 91,3 | 99,5 | 96,8 | 99,2 | 97,5 |
| **4** | 86.6 | 100,1 | 91,5 | 100,3 | 91,3 |
| **5** | 105.6 | 99,8 | 93,8 | 99,5 | 88,1 |
| **6** | 87.7 | 99,9 | 98,6 | 98,1 | 92,3 |
| **7** | 89,8 | 98,7 | 94,8 | 99,4 | 101,5 |
| **8** | 90.8 | 100,5 | 96,1 | 99 | 91,8 |
| **9** | 111,9 | 99,9 | 92,8 | 100,5 | 85,6 |
| **10** | 93,5 | 99,8 | 96,1 | 99,5 | 99,7 |
| **Average** | **93,8** | **99,6** | **95,0** | **99,4** | **94,5** |
| **SD** | **8,28** | **0,68** | **2,25** | **0,69** | **6,38** |
| **RSD%** | **8,83** | **0,68** | **2,37** | **0,70** | **6,75** |

The content uniformity evaluation showed that suspensions produced by Example 1 and Example 3 are more uniform suspensions with minimal standard deviations than commercial reference product. Reference product is resuspandable with difficulty and need some more shaking. The compositions of this invention will typically be redispersed in no more than 15 seconds with having improved content uniformity.

### Dissolution Profiles:

Selection of the dissolution medium to be used for dissolution testing is the most critical part of dissolution method development. In this invention, the formulation is a pH dependent pharmaceutical composition of levocloperastine fendizoate suspension which is less soluble at high pH and more soluble at low pH. Since there is no recorded dissolution medium, 0.1 N HCl dissolution medium was selected for dissolution tests.

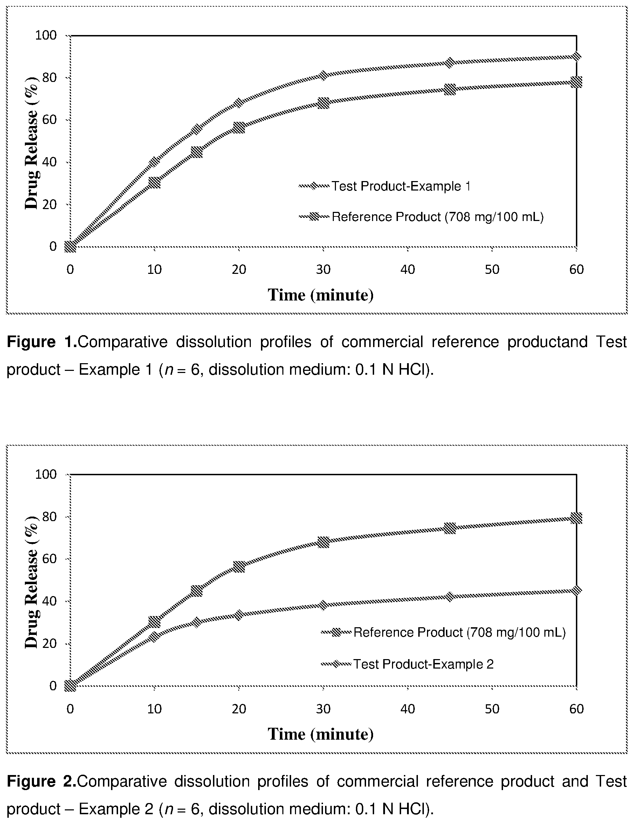

Comparative dissolution tests were conducted based on the general dissolution test method. The progress of dissolution is monitored for 60 minutes. Dissolution testing measures the portion (%) of levocloperastine fendizoate that has been dissolved in the dissolution medium. Dissolution test was performed with USP Apparatus-II, 0.1 N HCl, 50 rpm and 900 mL. The above exemplified compositions comprising levocloperastine fendizoate were tested in vitro and they were compared with a commercial reference suspension product. Figure 1 shows a comparative plot of the percentage of levocloperastine fendizoate dissolved as a function of time in each of the test samples prepared by Example-1 and commercial reference product.

Reference product releases about 65% of levocloperastine fendizoate in 30 minutes, whereas the pharmaceutical composition of the present invention (Example 1) releases about 80% of the levocloperastine fendizoate in 30 minutes. This significant increase in percent release of levocloperastine fendizoate leads to improved wettability, solubility, and hence increased percent release.

Figure 2 shows a comparative plot of the percentage of levocloperastine fendizoate dissolved as a function of time in each of the test samples prepared by Example-2 and commercial reference product.

It is well known that the particle size distribution of the drug substance have significant effects on final drug product performance (e.g. dissolution, bioavailability, content uniformity, stability, etc.). The dissolution rate of water-insoluble drugs such as levocloperastine fendizoate is strongly related to the particle size distribution. The specific surface area increased with decreasing particle size of the drug, resulting in an increase in dissolution rate. The drug release profile (Figure 2) of levocloperastine fendizoate having particle size distribution (D₉₀) higher than 150 µm shows lower dissolution rate with respect to reference product. The low dissolution rate results low bioavailability as well.

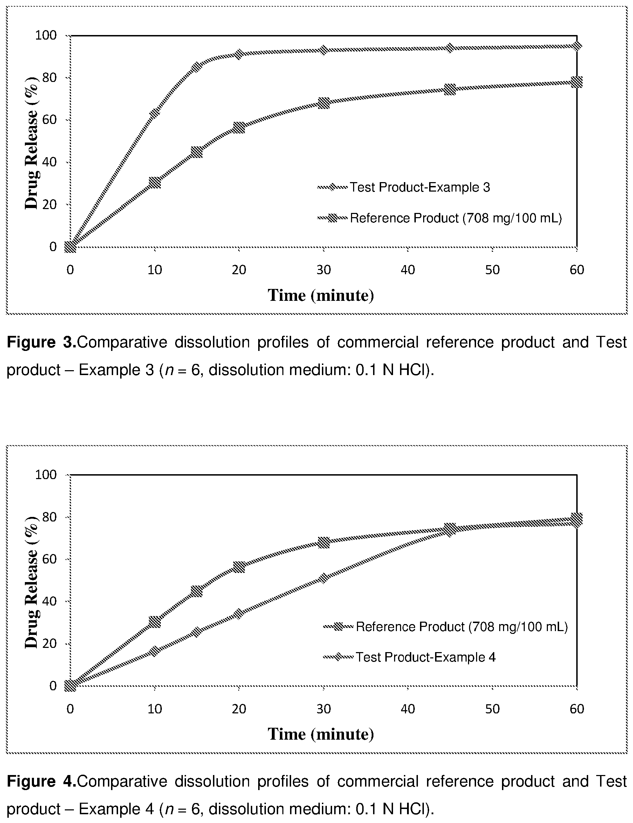

Figure 3 shows a comparative plot of the percentage of levocloperastine fendizoate dissolved as a function of time in each of the test samples prepared by Example-3 and commercial reference product.

As shown in Figure 3, the percent drug release increased with the decrease in the xanthan gum concentration. The pharmaceutical levocloperastine fendizoate suspension compositions demonstrate improved dissolution characteristics compared to the commercially available reference product.

The present inventors have noticed that the formulation of levocloperastine fendizoate suspension comprising levocloperastine fendizoate having particle size distribution (D₉₀) less than 100 µm, preferably less than 50 µm; a suspending system consisting essentially of 0.25% (w/v) to 0.36% (w/v) of xanthan gum; 0.25% of surfactant, namely polyoxyethyl stearate together results in significant increase in dissolution rate of the water-insoluble drug substance levocloperastine fendizoate.

Reference product releases about 65% of levocloperastine fendizoate in 30 minutes, whereas the pharmaceutical composition of the present invention (Example 3) releases about 85% of the levocloperastine fendizoate in 15 minutes with showing 'very rapidly dissolving' drug property. This significant increase in percent drug release of levocloperastine fendizoate is a surprising effect of this invention which leads to improved wettability, dissolution, and hence it would increase bioavailability.

Figure 4 shows a comparative plot of the percentage of levocloperastine fendizoate dissolved as a function of time in each of the test samples prepared by Example-4 and commercial reference product.

As shown in Figure 4, the percent drug release decreased with the decrease in the polyoxyethyl stearate amount in unit dose while xanthan gum concentration remains constant. This is because surfactants decrease the interfacial tension between drug particles and liquid thus liquid is penetrated in the pores of drug particle displacing air from them and thus ensures wetting. The low level use of polyoxyethyl stearate reduced the wettability of Levocloperastine fendizoate and caused a decrease in dissolution rate. Also, the low amount of surfactant deteriorates the redispersible property of produced suspension while causing the flocculation of the active substance.

**Table 3 Dissolution results (n = 6, dissolution medium: 0.1 N HCl)**

| **Time** | **Levocloperastine fendizoate % Release** | | | | |
|---|---|---|---|---|---|
| | **Reference product** | **Example 1** | **Example 2** | **Example 3** | **Example 4** |
| **15 min** | 45 | 56 | 30 | 85 | 26 |
| **30 min** | 68 | 81 | 38 | 93 | 51 |

### Stability Studies

Stability study of suspensions is a very important aspect to enable the patient to receive the intended amount of the drug(s) in the dose administered. Stability studies are required to be submitted by any applicant seeking approval for a new pharmaceutical product. Stability study requirements are covered, for example in the United States Pharmacopeia, in the Good Manufacturing Practices (GMP) as well as in FDA and ICH Guidelines. It is known that many drugs exhibit poor or modest shelf stability. The diminution of the concentration of a drag as a result of its degradation is inherently undesirable, as it makes therapy with the drug less certain. Stability issues are caused by environmental factors such as humidity, temperature and the like.

In the development of levocloperastine fendizoate suspension dosage form, stability was assessed under three different conditions (25°C ± 2°C, 30°C ± 2°C and 40°C ± 2°C) in temperature-programmable control cabinets. A temperature of 25°C ± 2°C/ 60 ± 5% RH (Relative Humidity) represents ambient temperature and carries out up to 24 months; 30°C ± 2°C/ /65 ± 5% RH represents intermediate temperature and carries out up to 12 months; 40°C ± 2°C/ 75 % ± 5 % RH represents extreme conditions and carries out up to 6 months.

Physical stability was defined as the absence of colour, odour and flavour changes and the evaluation of resuspension of solid phase by a reasonable amount of simple 15 seconds shaking. Physical stability is just as important as chemical stability. If the product does not resuspend adequately when shaken, the dose will be incorrect. The incomplete resuspension cause content uniformity problems. The initial doses removed will be low in doses. This occurs because drug remains at the bottom of the container preventing the correct concentration of the suspension when agitated. If part of the product is removed at a concentration significantly less than the labeled concentration, doses removed will become high in doses. This happens when further agitation removes drug from the bottom of the container and it is dispersed in the smaller volume of liquid.

In stability studies, the reference product needed about 1 minute shaking to become fully resuspended. It has been found that the present inventions (Example 1 & Example 3) improve the resuspendability characteristics of the suspension. The suspension produced with formulation Example 1 and Example 3 resuspended within 15 seconds shake. This resuspendability time is much lower than the shaking time required for the reference product. In order to test the content uniformity of stability samples, both suspended test and reference products were shaken for 15 seconds and a sample was removed from the suspension 2 cm below the liquid/air interface (6 times for each bottle) and assayed for the amount of levocloperastine fendizoate. The results proved the content uniformity problem of reference product while test products (Example 1 & Example 3) had assay results closer to the theoretical 100% of label and had less sample-to-sample variability.

The levocloperastine fendizoate suspensions of the invention (Example 1 & Example 3) are stable at three different conditions, in conventional packaging, e.g. glass bottles.

In addition to that, products prepared by Example 1 and Example 3 were tested for microbial contamination of total viable aerobic count, total combined yeast and mold count and Escherichia coli at regular intervals. Both of them passed microbial testing for 6 months period at 40°C ± 2°C, 75% ± 5 % RH extreme conditions.

### Bioequivalence study

As per USFDA guideline titled "Bioavailability and Bioequivalence Studies for Orally Administered Drug Products - General Considerations" Bioequivalence is defined as: "the absence of a significant difference in the rate and extent to which the active ingredient or active moiety in pharmaceutical equivalents or pharmaceutical alternatives becomes available at the site of drug action when administered at the same molar dose under similar conditions in an appropriately designed study."

The in-vivo studies were conducted in 36 healthy volunteers to assess the bioequivalence of the levocloperastine fendizoate oral suspension composition of the present invention (Example 1 - Levocloperastine fendizoate 708 mg/100 mL oral suspension; Batch No: 1505001) against commercial reference product as using an open-label, single dose, 2-period crossover, randomized design under fasting conditions.

Two drug products are considered 'bioequivalent' if they are pharmaceutical equivalents whose rate and extent of absorption are not significantly different when administered to patients or subjects at the same molar dose under similar experimental conditions and when 90% confidence interval (CI) for AUC and Cₘₐₓ fall within 80% to 125% for the test/ reference ratio.

Based on the results of the present study, extent (AUC₀₋ₜₗₐₛₜ) and rate (Cₘₐₓ) of bioavailability of levocloperastine from test preparation (Example 1- Levocloperastine Fendizoate 708 mg/100 ml Oral Suspension) and commercial reference product are well comparable. The 90 % confidence intervals for In-transformed AUC₀₋ₜₗₐₛₜ and Cₘₐₓ of levocloperastine meet the bioequivalence criteria of 80 % - 125 % therefore they became bioequivalent under fasting condition.

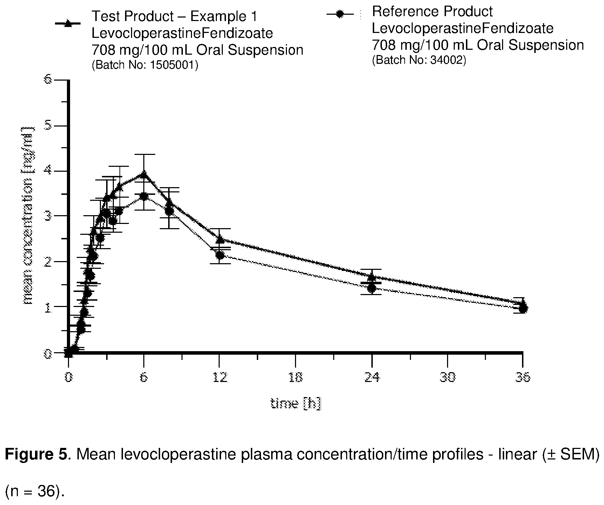

The present invention provides a formulation for oral pharmaceutically levocloperastine fendizoate suspension which has high similarity between in-vitro dissolution and in-vivo bioequivalence results. As shown in Figure 1, test product (Example -1) displays faster drug dissolution rates than the reference product as demonstrated from 0.1 N HCl dissolution medium, while at the same time mean levocloperastine plasma concentration/time profile (Figure 5) displays similar behavior with having faster drug plasma concentration rates.

In summary, based on the results presented herein, it is apparent that pharmaceutically acceptable oral suspension dosage form of an essentially water-insoluble drug substance of levocloperastine fendizoate is prepared with using the ingredient compositions and manufacturing methods discussed herein. Such compositions demonstrate improved drug dissolution rates and resuspendability as compared to reference product.

While the invention has been described in terms of its specific embodiments, certain modifications and equivalents will be apparent to those skilled in the art and are intended to be included within the scope of the invention.

### Explanation of figures:

**Figure 1****.** Comparative dissolution profiles of commercial reference product and Test product - Example 1 (n = 6, dissolution medium: 0.1 N HCl).
**Figure 2****.** Comparative dissolution profiles of commercial reference product and Test product - Example 2 (n = 6, dissolution medium: 0.1 N HCl).
**Figure 3****.** Comparative dissolution profiles of commercial reference product and Test product - Example 3 (n = 6, dissolution medium: 0.1 N HCl).
**Figure 4****.** Comparative dissolution profiles of commercial reference product and Test product - Example 4 (n = 6, dissolution medium: 0.1 N HCl).
**Figure 5****.** Mean levocloperastine plasma concentration/time profiles-linear (± SEM) (n = 36).

## Claims

1. An oral pharmaceutical suspension composition comprising:
(i) a therapeutic effective amount of levocloperastine fendizoate having a volume particle size distribution D₉₀ of less than 100 µm as measured by laser diffraction;
(ii) 0.25% (w/v) to 0.36% (w/v) of xanthan gum as a suspending agent;
(iii) 0.25% (w/v) of polyoxyethyl stearate as a surfactant; and
(iv) one or more pharmaceutically acceptable excipients.

2. An oral pharmaceutical suspension according to claim 1, wherein said drug substance has volume particle size distribution D₉₀ of less than 50 µm measured by laser diffraction.

3. An oral pharmaceutical suspension according to claim 1, wherein the composition exhibits a dissolution profile such that at least 85% of the levocloperastine fendizoate is released within 15 minutes; wherein the release rate is measured in Apparatus 2 (USP, paddle, 50 rpm) using 900 mLof 0.1 N HCl at 37°C ±0.5 °C.

4. An oral pharmaceutical suspension according to preceding claims, wherein pharmaceutically acceptable excipients comprises one or more of sweeteners, preservatives, flavoring agents, solvents.

5. An oral pharmaceutical suspension according to claim 4, wherein the sweeteners comprise one or more of sucrose, sorbitol, xylitol, dextrose, fructose, maltitol, acesulfame potassium, aspartame, saccharin, saccharin sodium, liquid maltitol, liquid glucose, cyclamate, sodium cyclamate.

6. An oral pharmaceutical suspension according to claim 4, wherein the preservatives comprise one or more of sodium benzoate, benzoic acid, ethylenediamine tetraacetic acid, sorbic acid, bronopol, butyl paraben, methyl paraben, ethyl paraben, propyl paraben, sodium propionate, chlorhexidine, potassium sorbate, propylene glycol, sodium bisulfite, sodium metabisulfite, sodium salts of hydroxybenzoate.

7. An oral pharmaceutical suspension according to claim 4, wherein the flavoring agents comprise one or more of artificial strawberry flavor, artificial cream flavor, vanilla, cherry, raspberry.

8. An oral pharmaceutical suspension according to claim 4, wherein the solvents comprise one or more of water, glycerol, propylene glycol, polyethylene glycol, ethanol.

## Patentansprüche

1. Orale pharmazeutische Suspensionszusammensetzung, umfassend
(i) eine therapeutisch wirksame Menge von Levocloperastinfendizoat mit einer Volumenteilchengrößenverteilung D90 von weniger als 100 µm, gemessen durch Laserbeugung;
(ii) 0,25% (gew/vol) bis 0,36% (gew/vol) Xanthangummi als Suspendiermittel; und
(iii) 0,25% (gew/vol) Polyoxyethylstearat als oberflächenaktives Mittel; und
(iv) einen oder mehrere pharmazeutisch annehmbare Hilfsstoffe.

2. Orale pharmazeutische Suspension nach Anspruch 1, wobei der Arzneistoff eine Volumenpartikelgrößenverteilung D90 von weniger als 50 µm, gemessen durch Laserbeugung, aufweist.

3. Orale pharmazeutische Suspension nach Anspruch 1, wobei die Zusammensetzung ein solches Auflösungsprofil aufweist, dass mindestens 85% des Levocloperastin-Fendizoats innerhalb von 15 Minuten freigesetzt werden, wobei die Freisetzungsrate in Apparat 2 (USP, Paddle, 50 U/min) unter Verwendung von 900 ml 0,1 N HCl bei 37°C ± 0,5°C gemessen wird.

4. Orale pharmazeutische Suspension nach einem der vorhergehenden Ansprüche, wobei pharmazeutisch annehmbare Hilfsstoffe einen oder mehrere Süßstoffe, Konservierungsmittel, Aromastoffe, Lösungsmittel umfassen.

5. Orale pharmazeutische Suspension nach Anspruch 4, wobei die Süßungsmittel eines oder mehrere von Saccharose, Sorbit, Xylit, Dextrose, Fructose, Maltit, Acesulfam-Kalium, Aspartam, Saccharin, Saccharin-Natrium, flüssigem Maltit, flüssiger Glucose, Cyclamat, Natriumcyclamat umfassen.

6. Orale pharmazeutische Suspension nach Anspruch 4, wobei die Konservierungsmittel eines oder mehrere von Natriumbenzoat, Benzoesäure, Ethylendiamintetraessigsäure, Sorbinsäure, Bronopol, Butylparaben, Methylparaben, Ethylparaben, Propylparaben, Natriumpropionat, Chlorhexidin, Kaliumsorbat, Propylenglykol, Natriumbisulfit, Natriummetabisulfit und Natriumsalzen von Hydroxybenzoat umfassen.

7. Orale pharmazeutische Suspension nach Anspruch 4, wobei die Geschmacksstoffe einen oder mehrere der folgenden Geschmacksstoffe umfassen: künstliches Erdbeeraroma, künstliches Sahnearoma, Vanille, Kirsche, Himbeere.

8. Orale pharmazeutische Suspension nach Anspruch 4, wobei die Lösungsmittel eines oder mehrere der folgenden umfassen: Wasser, Glycerin, Propylenglycol, Polyethylenglycol, Ethanol.

## Revendications

1. Composition de suspension pharmaceutique orale comprenant: Composition de suspension pharmaceutique orale comprenant:
(i) une quantité thérapeutiquement efficace de fendizoate de lévocloperastine dont la distribution de la taille des particules en volume D90 est inférieure à 100 µm, mesurée par diffraction laser,
(ii) 0,25% (p/v) à 0,36% (p/v) de gomme xanthane comme agent de suspension,
(iii) 0,25% (p/v) de stéarate de polyoxyéthyle comme agent tensioactif,
(iv) un ou plusieurs excipients pharmaceutiquement acceptables.

2. Suspension pharmaceutique orale selon la revendication 1, dans laquelle ladite substance médicamenteuse présente une distribution volumétrique de la taille des particules D90 inférieure à 50 µm, mesurée par diffraction laser.

3. Suspension pharmaceutique orale selon la revendication 1, dans laquelle la composition présente un profil de dissolution tel qu'au moins 85% du fendizoate de lévocloperastine est libéré en 15 minutes; la vitesse de libération est mesurée dans l'appareil USP (palette, 50 rpm) en utilisant 900 ml de HCl 0,1 N à 37±0,5°C.

4. Suspension pharmaceutique orale selon les revendications précédentes, dans laquelle les excipients pharmaceutiquement acceptables comprennent un ou plusieurs édulcorants, des conservateurs, des agents aromatiques et des solvants.

5. Suspension pharmaceutique orale selon la revendication 4, dans laquelle les édulcorants comprennent un ou plusieurs des éléments suivants: saccharose, sorbitol, xylitol, dextrose, fructose, maltitol, acésulfame de potassium, aspartame, saccharine, saccharine sodique, maltitol liquide, glucose liquide, cyclamate, cyclamate de sodium.

6. Suspension pharmaceutique orale selon la revendication 4, dans laquelle les conservateurs comprennent un ou plusieurs des éléments suivants: benzoate de sodium, acide benzoïque, acide éthylènediamine tétraacétique, acide sorbique, bronopol, parabène de butyle, parabène de méthyle, parabène d'éthyle, parabène de propyle, propionate de sodium, chlorhexidine, sorbate de potassium, propylène glycol, bisulfite de sodium, métabisulfite de sodium, sels sodiques d'hydroxybenzoate.

7. Suspension pharmaceutique orale selon la revendication 4, dans laquelle les agents aromatisants comprennent un ou plusieurs arômes artificiels de fraise, de crème artificielle, de vanille, de cerise, de framboise.

8. Suspension pharmaceutique orale selon la revendication 4, dans laquelle les solvants comprennent un ou plusieurs des éléments suivants: eau, glycérol, propylène glycol, polyéthylène glycol, éthanol.
